(19) Europäisches Patentamt
European Patent Office
Office européen des brevets

(11) **EP 2 051 620 B1**

(12) **EUROPEAN PATENT SPECIFICATION**

(45) Date of publication and mention
of the grant of the patent:
**28.04.2010 Bulletin 2010/17**

(21) Application number: **07825964.5**

(22) Date of filing: **26.07.2007**

(51) Int Cl.:
**A61B 5/00** (2006.01)

(86) International application number:
**PCT/IB2007/052971**

(87) International publication number:
**WO 2008/017981 (14.02.2008 Gazette 2008/07)**

(54) **METHOD AND DEVICE FOR MONITORING A PHYSIOLOGICAL PARAMETER**

VERFAHREN UND VORRICHTUNG ZUR ÜBERWACHUNG EINES PHYSIOLOGISCHEN PARAMETERS

PROCÉDÉ ET DISPOSITIF POUR SURVEILLER UN PARAMÈTRE PHYSIOLOGIQUE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **08.08.2006 EP 06118605**

(43) Date of publication of application:
**29.04.2009 Bulletin 2009/18**

(73) Proprietor: **Koninklijke Philips Electronics N.V.**
**5621 BA Eindhoven (NL)**

(72) Inventors:
• **VERBITSKY, Evgeny**
  **NL-5656 AE Eindhoven (NL)**
• **DE BOKX, Pieter, K.**
  **NL-5656 AE Eindhoven (NL)**
• **DENTENEER, Theodorus, J., J.**
  **NL-5656 AE Eindhoven (NL)**

• **VON BASUM, Golo**
  **NL-5656 AE Eindhoven (NL)**
• **OTAL, Begonya**
  **NL-5656 AE Eindhoven (NL)**
• **MURESAN, Adrian, S.**
  **NL-5656 AE Eindhoven (NL)**
• **GORIS, Annelies**
  **NL-5656 AE Eindhoven (NL)**
• **VAN HERK, Johannes, J.**
  **NL-5656 AE Eindhoven (NL)**

(74) Representative: **Van Velzen, Maaike Mathilde**
**Philips**
**Intellectual Property & Standards**
**P.O. Box 220**
**5600 AE Eindhoven (NL)**

(56) References cited:
**EP-A- 1 078 258        WO-A-97/28737**
**US-A1- 2005 240 356     US-B1- 6 923 763**

**Description**

FIELD OF THE INVENTION

The present invention relates to a method and device for monitoring a physiological parameter.

BACKGROUND OF THE INVENTION

About two hundred million people suffer from diabetes mellitus. Diabetes is a chronic condition in which the body does not properly regulate or utilize the insulin, which results in abnormal (too high/too low) levels of glucose in the blood. A tight glycemic monitoring is desirable for these patients. Presently, methods for observing the glucose levels require, for example, the finger-stick blood sample, which is further processed by a glucose meter. But observation alone is not sufficient for glycemic control. Any diabetic decision support system should be able to predict future values of the blood glucose with a time horizon of 30 to 60 minutes as accurately as possible. Within the predicted time the patient can then take the appropriate actions, as for example food intake or insulin injection, so that theses actions can become effective in time.

There are several problems which must be resolved by a prediction method. First of all, the dynamics of glucose - insulin interaction is rather complicated. Even continuous measurement of the blood glucose provides only part of the actual information about the state of the system. Secondly, fluctuations in blood glucose levels are intrinsically non-stationary and depend on external factors like food and insulin injections. Thirdly, even for the same patient, given the same food intake and insulin injections , the glucose dynamics can be different due to variation in a large number of parameters, as for example body temperature or physical activity, which are known to affect the glucose metabolic rates.

For example, in US 6 923 763 B1 a prediction method is proposed based on a complex dynamic model that includes measured glucose values, heart rate and heart rate variability to try to estimate the blood glucose level in the future and estimate the risk of hypoglycemia. The approach is based on dynamic modeling of the glucose-regulation. One drawback of the prediction by dynamical models for glucose evolution is that the models are non-linear, complex and contain a large number of physiological parameters which cannot be measured.

SUMMARY OF THE INVENTION

It is therefor an object of the present invention to provide a method for monitoring a physiological parameter with a more reliable prediction of the evolution of the physiological parameter.

The above objective is accomplished by a method for monitoring a physiological parameter, in particular for monitoring a blood glucose level, comprising

-    logging the physiological parameter by repeated measurements and predicting the evolution of the physiological parameter,

wherein the evolution of the physiological parameter over an interval of autonomous evolution is predictable by a parametric model.

The logging comprises preferably a non-invasive, continuous method of measuring the physiological parameter, e.g. for glucose measurements. Continuous in this sense means, that, if required, the measurements are performed about every 5 to 10 minutes, preferably automatically.

An interval of autonomous evolution in the sense of this invention is meant to be a time interval with a relatively simple evolution of the physiological parameter, e.g. the blood glucose level. Such intervals are primarily characterized by the absence of drastic external influences like food intake, insulin injections, physical exercises, etc. Within an interval of autonomous evolution one is able to use a parametric model for the physiological parameter, like glucose concentration. The person skilled in the art understands that the parametric model can be derived from statistical analysis of continuous traces of the evolution of the physiological parameter, which can be described well approximated over a limited time interval. An advantage of the inventive method is, that a limited number of parameters and models of reduced complexity can be used, despite the complexity of underlying processes.

Within an autonomous interval, the evolution of the physiological parameter is predictable by the following parametric model:

$$G(t) = Q(t; p_1, \dots, p_k),$$

where G(t) is the level of the physiological parameter at time t, which is predicted by the value Q at time t and with the parameters $p_l$, ..., $p_k$. The parametric model is used to extrapolate, i.e., predict the future values of the physiological parameter, especially the blood glucose concentration.

For the interval of autonomous evolution, which comprises a plurality of previous measurements (at times $t_{k-i}$, for i=1...m), an optimal set of parameters or parameter vector $\vec{p}_{k-1}$, according to the parametric model is preferably determined by the Least Squares Method:

$$\vec{p}_{k-1} = \underset{\vec{p}}{\operatorname{argmin}} \sum_{i=1}^{m} \left| G(t_{k-i}) - Q(t_{k-i}; \vec{p}) \right|^2$$

The prediction according to the inventive method comprises the steps of

- adding a new measurement of the physiological parameter to the previous measurements of the interval of autonomous evolution,
- conducting a test, whether the new measurement together with the previous measurements still form an interval of autonomous evolution, and depending upon this test,
- adapting the interval of autonomous evolution and preferably adapting the parametric model,
- predicting the evolution of the physiological parameter, using the parametric model for the adapted interval of autonomous evolution and
- associating a likelihood value to the predicted evolution of the physiological parameter, the prediction of the evolution of the physiological parameter being discarded (23) if the likelihood value is below a certain threshold value.

A new measurement $(t_k, G(t_k))$ and a multitude of previous measurements of the interval of autonomous evolution $\{(t_{k-i}, G(t_{k-i}))\}$, with i running from 1 to m, together form a new interval upon which the test is conducted. The person skilled in the art understands, that in this test, the new measurement is compared to the previously predicted evolution of the physiological parameter. The interval of autonomous evolution is then adapted accordingly. For example the interval of autonomous evolution is narrowed such that the parametric model is able to predict the evolution of the physiological parameter, i.e. to predict the new measurement. The person skilled in the art also understands that the modified interval of autonomous evolution comprises at least the new measurement. When the interval of autonomous evolution is adapted, the parametric model is preferably adapted as well. The adapted parametric model is then used for the prediction of the future evolution of the physiological parameter.

The future evolution, using the optimal set of parameters, is predicted by the parametric model:

$$\tilde{G}(t_k + \Delta t) = Q(t_k + \Delta t; \vec{p}_k),$$

where $\Delta t$ is the desired time horizon of 30 to 60 minutes.

Advantageously, the inventive parametric model, in principle, is not physical, i.e. the parametric model is primarily selected on the basis of adequate description of glucose traces, and not necessarily using complex models describing the full glucose regulatory system. Nevertheless, incorporation of at least parts of physical models is not excluded by the present invention. It is beneficial to use non-physical models, because they are simpler and contain fewer or no unknown parameters. For a complex non-linear dynamical system, which does not admit an analytic solution, proper estimation of the unknown parameters is much more difficult, if not infeasible. This can be done for type 1 diabetes, which is easier to model than type 2 or type 3 diabetes. The inventive method is advantageously applicable to glucose monitoring and prediction in general, and does not assume a particular type of diabetes.

Advantageously, a likelihood value is associated to the predicted evolution of the physiological parameter, the prediction of the evolution of the physiological parameter being discuded if the likelihood value is below a certain, predefined threshold value. The likelihood value advantageously reflects the trust in the accuracy of the prediction. For example it the interval of autonomous evolution used for the prediction of glucose concentration is too small (too short), or, the interval was just adapted, this reduces the likelihood value. On the other hand, if the interval of autonomous evolution is relatively long and the set of optimal parameters has not changed drastically in a few last iterations, the likelihood value is higher. An advantage of the embodiment is to avoid prediction at all if the likelihood value is too small. The primary goal of the inventive monitoring and prediction of, for example, glucose concentrations is to prevent hypoglycemic and hyperglycemic events, is events where glucose levels become dangerously low or dangerously high.

These events typically occur at the end of long intervals of autonomous evolution. Hence, according to the present invention such predictions will have high likelihood values. Advantageously, extremely low or high glucose predictions cause an alarm for the patient and call for an appropriate action and a large number of false alarms is prevented.

The interval of autonomous evolution is preferably adapted by removing at least one measurement of the plurality of measurements of the interval of autonomous evolution. The person skilled in the art understands, that the oldest measurements of the interval of autonomous evolution are preferably removed first. The advantage of this method is, that an interval of autonomous evolution is not terminated, once the test is negative, but prediction is continued with a modified, smaller interval of autonomous evolution.

In a preferred embodiment of the present invention the test is conducted again, now testing whether the new measurement together with the measurements of the adapted interval form an interval of autonomous evolution. Furthermore preferred, the steps of removing at least one measurement and conducting the test is repeated until the adapted interval is an interval of autonomous evolution.

A preferred way of obtaining a new interval of autonomous evolution is, to repeatedly remove only the oldest measurement and then conduct the test, so that the longest possible, new interval of autonomous evolution can be determined. If, however, the plurality of measurements fails the test until only the new measurement is left, no interval of autonomous evolution can be determined. According to the inventive method, a new interval of autonomous evolution can be determined earliest after the next measurement.

A number of quantitative characteristics can be used to evaluate the quality of the new interval of autonomous evolution. Preferably, in the test an error is calculated by comparing the physiological parameter of the new measurement to a predicted physiological parameter of a preceding iteration:

$$c_k = \left| G(t_k) - Q(t_k; \vec{p}_{k-1}) \right|$$

If the error does not exceed a predefined threshold, the interval of autonomous evolution is extended by adding a new observation value.

In another preferred embodiment, a new set of parameters for the parametric model is determined, wherein the parametric model with the new set of parameters optimally describes the evolution of the physiological parameter over the interval of autonomous evolution including the new measurement.

The new set of parameters is preferably determined after the Least Squares Method:

$$\vec{p}_k = \operatorname*{argmin}_{\vec{p}} \sum_{i=0}^{m} \left| G(t_{k-i}) - Q(t_{k-i}; \vec{p}) \right|^2$$

Then, a distance between a new vector, defined by the new set of parameters and a former vector, defined by the set of parameters of the preceding iteration is calculated:

$$\left\| \vec{p}_k - \vec{p}_{k-1} \right\|^2 = \sum_{i=1}^{m} \left| p_{k,i} - p_{k-1,i} \right|^2$$

Again, if the distance does not exceed a predefined threshold value, the new measurement is added and the current interval of autonomous evolution is extended.

If the measurement is rejected in the test, the current interval of autonomous evolution is adapted. Most advantageously the new measurement can be added to the plurality of measurements forming the interval of autonomous evolution on the right, and the oldest measurements can be removed from the left until a smaller plurality of measurements meets the criterion of autonomous evolution.

In a preferred embodiment of the inventive method, a step of alarming a patient in case the predicted evolution of the physiological parameter is below or above a dangerous level. Advantageously, a patient suffering from diabetes can be alarmed early enough to prevent hypoglycemia or hyperglycemia.

Alternatively or additionally, an advice for the patient is provided, suggesting an agent or an amount of an agent to be administered, in particular an amount of insulin. The inventive system advantageously provides a decision support to the patient.

An important aspect of the prediction is the stability of the results. Variations in the predicted evolution, which are faster than typical variations of glucose concentrations, should be avoided. In a preferred embodiment of the invention, the step of predicting the evolution of the physiological parameter is conducted using a multitude of parametric models for the adapted interval of autonomous evolution and obtaining the prediction as a weighted sum of the predictions of the multitude of parametric models:

$$\mathrm{Prediction}(t + \Delta t) = \sum_{j=1}^{M} w_j \mathrm{Pred.Model}_j(t + \Delta t)$$

wherein the weights $w_j$ of the parametric models are selected adaptively, preferably depending on a quality of the prediction of each parametric model in the previous iterations and/or depending on the number of iterations each parametric model has been used. For example, the weight $w_j$ for the j-th parametric model reflects the quality of the prediction of the j-th model demonstrated so far and/or the time period the model during which the model has been used. If the weight of a particular parametric model drops below a certain predefined threshold value the parametric model is preferably discarded (or its weight is set to zero) and a substitute parametric model is added to the multitude of parametric models. One of the advantages of this embodiment is to increase the stability of the prediction. Machine Learning theory provides methods for collecting predictions from several models ("experts") in one super-model ("super-expert"), which can outperform each of the individual "experts".

Preferably a number of threshold values, particularly the threshold value for the weight of the parametric models and/or the threshold for the likelihood value are adapted. Advantageously, the variability between different individuals is met by this embodiment. Again, Machine Learning techniques may advantageously be used, to adapt the threshold values.

In a further preferred embodiment the inventive method comprises the step of adapting the interval of autonomous evolution and/or the parametric model depending on external parameters known to be affecting the physiological parameter. The external parameters are preferably associated to ingestion, administration of insulin, physical activity and/or emotional stress of the patient. These major factors, which influence the intervals of autonomous evolution in glucose dynamics, are preferably detected and the parametric model is adapted appropriately. This advantageously enhances the accuracy of the predicted evolution, as the intervals of autonomous evolution can be updated earlier, for example.

In a still further preferred embodiment, the external parameters are manually inputted by a patient or by an auxiliary. For example, a device can be equipped with "food", "insulin", "exercise" buttons, which will advantageously provide information on forthcoming changes in the interval of autonomous evolution. Alternatively calorie input forms, food lists and/or databases can be used.

According to another preferred embodiment at least one of the external parameters is detected by at least one sensor. Sensors could be heart rate sensors, heart rate variability sensors, insulin pump sensors or food intake sensors. The reliability of the prediction is advantageously enhanced, as it does not depend on the accurateness of the input of the patient, who may forget to give or give wrong input on food intake, exercise or amounts of injected insulin.

In a preferred embodiment the physical activity is determined by an accelerometer, more preferably by an accelerometer summarizing the acceleration in three directions in space. In a further preferred embodiment the physical activity is determined by analyzing a heart rate of a patient by a heart rate sensor. With relation to exercise, these embodiments advantageously provide relatively accurate readings for a parameter which is difficult to quantify for a patient.

Another aspect of the present invention is a monitoring device for monitoring a physiological parameter of a patient, in particular for monitoring a blood glucose level, comprising a physiological parameter measuring means and a computing means, the computing means having a data storage for storing measurements and a processor unit for calculating a prediction of the evolution of the physiological parameter, using the method according to the present invention. The monitoring device advantageously provides reliable prediction of the evolution of the physiological parameter.

The monitoring device preferably comprises input means for inputting external parameters which are known to affect the physiological parameter, such as ingestion, administration of insulin, physical activity and/or emotional stress of the patient. The input means preferably comprises at least a button for manual input of each external parameter. In this embodiment the quality of the predicted evolution is enhanced, as information on forthcoming changes in the interval of autonomous evolution is provided by the patient.

Preferably the monitoring device comprises output means for outputting alarm messages and/or advice messages to the patient. The inventive monitoring device thus provides a (diabetic) decision support system with the ability to predict future evolution of the physiological parameter, especially the blood glucose level, with a time horizon of 30 to 60 minutes.

Preferably the monitoring device being connected to a detector for detecting a physical activity of the patient, preferably a three-axial accelerometer.

It is known that food intake, insulin uptake and exercise influence the blood glucose level most. The reliability of the prediction of the monitoring device usually depends on the accurateness of the input of the user. If he or she forgets to give or gives wrong input on food intake, exercise or amounts of injected insulin the model/device will give false predictions. With relation to exercise it is difficult anyhow to quantify the input. The monitoring device according to the present invention overcomes the abovementioned disadvantages by providing a monitoring device that comprises an detector for detecting a physical activity of the patient. This activity sensor senses the motions of a body and therewith provides a continuous input with relation to the physical activity of a person suffering from diabetes. In this way the input with regard to the exercise level is advantageously reliable.

The detector is preferably wearable by the patient, more preferably the detector is arranged in or at a belt, a watch or a mobile phone. This makes it easy to wear the detector all day long, which advantageously provides objective and detailed information on the duration and intensity of the activity of the patient (sleep, rest, sitting, exercise etc).

Preferably, the monitoring device in combination with the detector is initialized once to learn the specific reaction of a patient's activity on his or her blood glucose level, also in relation to prior food- and insulin-intake. Preferably, a first time usage requires an individual initialization of the model. For example, the user wears the detector together with the monitoring device and performs a set of standardized activities of different intensities. The prediction of the evolution of the physiological parameter is adapted during this initialization phase on an individual basis of the response of the different activity levels on the physiological parameter, in particular the blood glucose level. This information is used to update the parameter prediction model. An existing blood glucose level prediction device can be extended with the detector to improve the reliability of the blood glucose level evolution prediction.

BRIEF DESCRIPTION OF THE INVENTION

These and other characteristics, features and advantages of the present invention will become apparent from the following detailed description, taken in conjunction with the accompanying drawings, which illustrate, by way of example, the principles of the invention. The description is given for the sake of example only, without limiting the scope of the invention. The reference figures quoted below refer to the attached drawings.

Fig. 1 depicts exemplarily a blood glucose evolution over an interval of time.
Fig. 2 illustrates schematically a method according to the present invention.
Fig. 3 illustrates schematically the method and embodiments of the monitoring device according to the present invention.
Fig. 4 depicts exemplarily a diagram of physical activity over an interval of time.
Fig. 5 illustrates an idealized pattern of insulin secretion over an interval of time.

DETAILED DESCRIPTION OF THE EMBODIMENT

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes.

Where an indefinite or definite article is used when referring to a singular noun, e.g. "a", "an", "the", this includes a plural of that noun unless something else is specifically stated.

Furthermore, the terms first, second, third and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described of illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the present description and claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

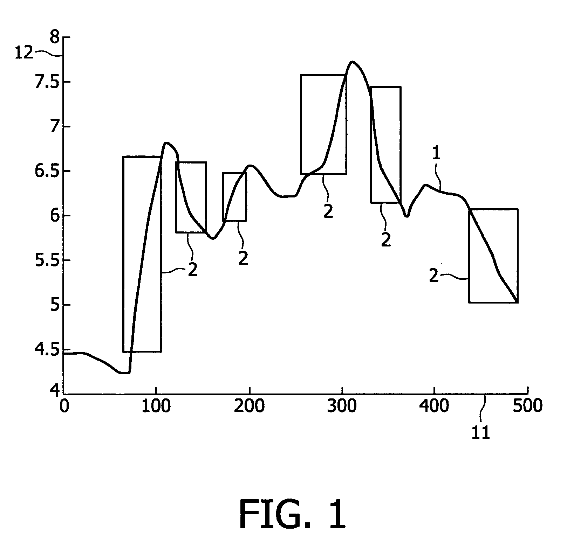

Fundamental work on glucoregulatory systems demonstrate without a doubt a great variation of possible dynamic evolutions of the blood glucose level 1, one of which is exemplarily depicted in Figure 1. The abscissa 11 shows an interval of time in minutes and the axis of ordinates 12 the blood glucose concentration in milligrams per deciliter. Statistical analysis of continuous glucose traces 1 allows, that despite the complexity of underlying processes, glucose traces 1, limited in time, can be described and well approximated, using a limited number of parameters and models of reduced complexity. By inspecting glucose trace 1, it is possible to identify time intervals 2 with a relatively simple glucose evolution. Such time intervals 2 are referred to in here as intervals of autonomous evolution 2. Such intervals are primarily characterized by an absence of drastic external influences like food intake, insulin injections, physical exercise. Within an interval of autonomous evolution 2 it is possible to use a relatively simple parametric model for the glucose concentration.

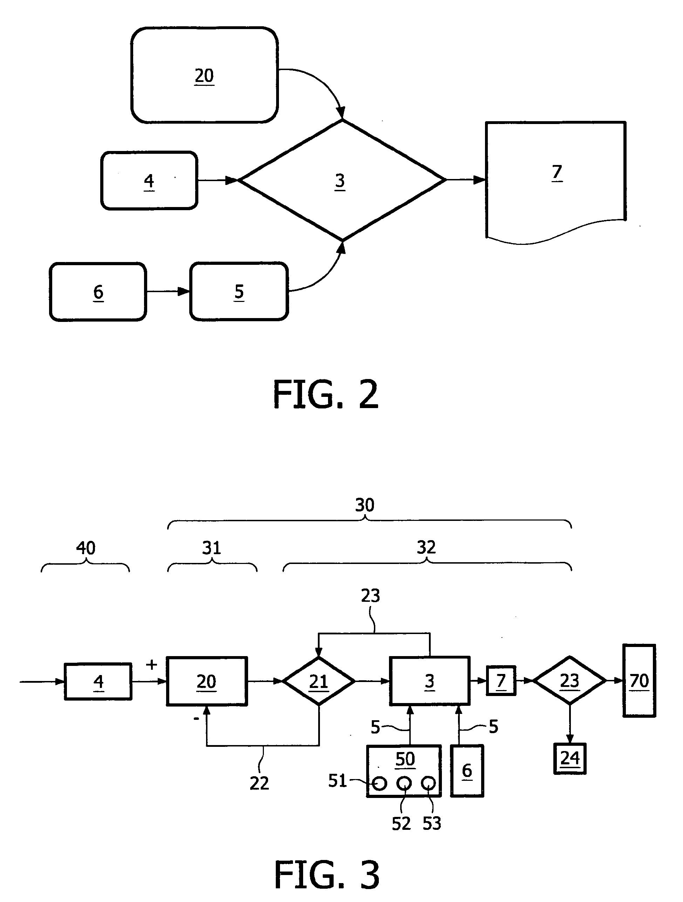

In Figure 2 the method according to the invention is depicted schematically. By analyzing a multitude of measurements, a parametric model 3 is created, which is able give a predicted evolution 7 for the physiological parameter with sufficient accuracy over an interval of autonomous evolution 2. Besides a new measurement 4 and stored previous measurements 20, no physical parameters are necessary for the inventive method. In order to enhance the prediction 7, the parametric model can incorporate external parameters 5, which arc known to influence the physiological parameter. For example ingestion, insulin administration, physical activity or mental stress radically influence the blood glucose level. An external parameter 5 can be inputted manually or automatically by a sensor or detector 6, as for example a detector for the physical activity, such as a heart rate sensor or an accelerometer.

In Figure 3 an embodiment of the inventive method is schematically depicted, further showing the appropriate parts of the inventive monitoring device. A measurement device 40 is used to take the new measurement 4, which is added to a plurality of former measurements, stored on a data storage 31. The plurality of former measurements represent the former interval of autonomous evolution 2 at the time of a previous measurement. The new measurement 4 and the plurality of former measurements form together a new plurality of measurements 20. A test 21 is conducted, whether the new plurality of measurements 20 forms an interval of autonomous evolution. For the test 21, the former prediction of the future evolution by the parametric model 3 is compared to the new measurement 4, which is shown by feedback line 23. If the criterion of an interval of autonomous evolution is not met, at least one, preferably the oldest measurement 22 of the plurality of measurements 20 is removed and the test 21 is repeated. If the criterion of an interval of autonomous evolution is met, the parametric model 3 is adapted if necessary and a prediction 7 for the future evolution of the physiological parameter is calculated. A likelihood value 23, representing the reliability of the predicted evolution 7, is also calculated. If the likelihood value 23 is below a predefined threshold, the prediction 7 is discarded 24. Otherwise the predicted evolution 7 is outputted by an output means 70. The process starts again upon the next new measurement. All mentioned calculations are performed by a processor unit 32, which is, like data storage 31, part of a computing means 30.

According to an embodiment of the invention, the parametric model 3 is additionally adapted depending upon external parameters 5, which are derived from an input means 50 and/or from a detector 6. The input means 50 is preferably equipped with buttons 51, 52, 53, which are pressed by a patient upon certain activities in order to provide information on forthcoming changes in the interval of autonomous evolution to the monitoring device. For example button 51 is used for ingestion, button 52 for insulin administration and button 53 for physical exercises. Detector 6 preferably detects physical activity of the patient automatically, for example by way of a three-axial accelerometer worn by the patient.

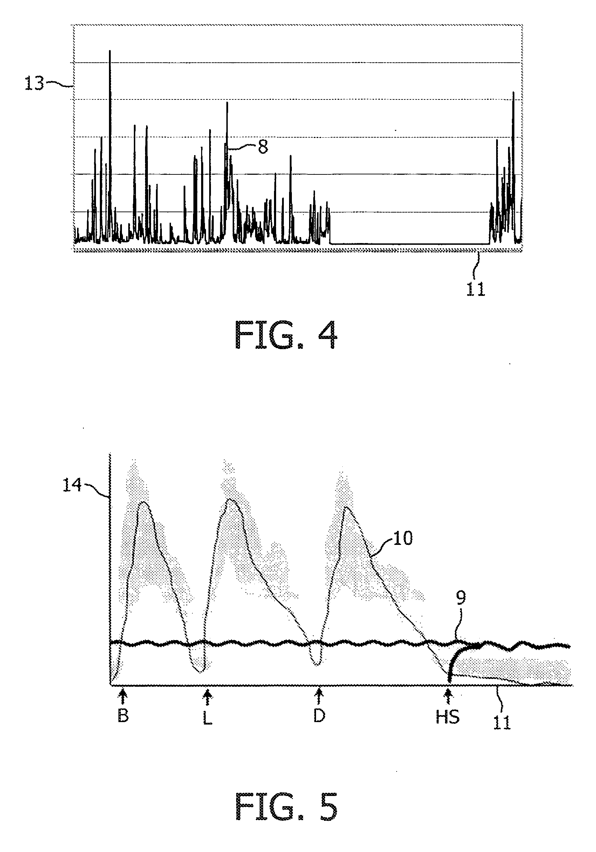

Figure 4 shows a diagram of a detected activity pattern 8 for a person who wore the detector 6 for 24 hours. The time is shown on the abscissa 11. For example a tri-axial accelerometer gives every minute the sum of accelerations in three directions. These accelerations are shown on the axis of ordinates. They can be converted into a calorific value, but the arbitrary units can as well be directly used by the parametric model.

Figure 5 shows an idealized pattern of insulin secretion for a healthy individual who has consumed three standard meals, breakfast B, lunch L and dinner D. Again on the abscissa 11 the time is shown. On the ordinate the insulin effect is qualitatively shown. HS marks the bedtime. People with diabetes can use two types of insulin; the long acting insulin or basal insulin 9 and the short acting insulin 10 (bolus). The bolus type of insulin 9 is mostly injected before the onset of a meal. The effects of the long acting insulin 10 can be predicted more accurately by the inventive method and monitoring device. A day of less activity or of higher activity than normal, influences the insulin level, which may result in hyperglycemia or hypoglycemia. The detector for detecting physical activity measures and summarizes the acceleration, compares it to a day of normal activity and the monitoring system can warn the patient that he/she is more/less active and should adapt his insulin profile.

The present invention is related to a method and device for monitoring a physiological parameter, like the blood glucose level, using prediction of future evolution of the physiological parameter based on continuous traces. The present method and device can be employed as a decision support system for diabetic patients.

## EP 2 051 620 B1

**Claims**

1. Method for monitoring a physiological parameter (1), in particular for monitoring a blood glucose level, comprising

    - logging the physiological parameter (1) by repeated measurements (4) and
    - predicting the evolution of the physiological parameter (1),

    wherein the evolution of the physiological parameter over an interval of autonomous evolution (2) is predictable by a parametric model (3), the prediction comprising the steps of

    - adding a new measurement (4) of the physiological parameter to the previous measurements (20) of the interval of autonomous evolution (2),
    - conducting a test (21), whether the new measurement together with the previous measurements still form an interval of autonomous evolution and, depending upon this test,
    - adapting the interval of autonomous evolution and preferably adapting the parametric model (3),
    - predicting (7) the evolution of the physiological parameter, using the parametric model (3) for the adapted interval of autonomous evolution and

    associating a likelihood value to the predicted evolution of the physiological parameter, the prediction of the evolution of the physiological parameter being discarded (23) if the likelihood value is below a certain threshold value.

2. Method according to claim 1, wherein the interval of autonomous evolution (2) is adapted by removing at least one measurement (22) of a plurality of measurements of the interval of autonomous evolution.

3. Method according to claim 2, wherein the test is conducted, whether the new measurement together with the measurements of the adapted interval form an interval of autonomous evolution.

4. Method according to claim 3, wherein the steps of removing at least one measurement (22) and conducting the test is repeated until the adapted interval is an interval of autonomous evolution.

5. Method according to one of claims 1 or 3, wherein the step of conducting the test comprises

    - calculation of an error by comparing the physiological parameter of the new measurement to a predicted physiological parameter of a preceding iteration, or
    - determination of a new set of parameters for the parametric model, wherein the parametric model with the new set of parameters optimally describes the evolution of the physiological parameter over the interval of autonomous evolution including the new measurement and calculation of a distance between a new vector, defined by the new set of parameters and a former vector, defined by a set of parameters of a preceding iteration.

6. Method according to claim 1, further comprising

    - the step of alarming a patient in case the predicted evolution (7) of the physiological parameter (1) is below or above a dangerous level and/or
    - the step of outputting an advice, in particular suggesting an agent or an amount of an agent to be administered, in particular an amount of insulin.

7. Method according to claim 1, the step of predicting the evolution of the physiological parameter is conducted using a multitude of parametric models for the adapted interval of autonomous evolution and obtaining the prediction as a weighted sum of the predictions of the multitude of parametric models, wherein the weights of the parametric models are defined adaptively, preferably depending upon a quality of the prediction of each parametric model in the previous iterations and/or depending upon the number of iterations each parametric model has been used.

8. Method according to claim 7, wherein each parametric model is discarded when its weight drops below a threshold value and a substitute parametric model is added to the multitude of parametric models.

9. Method according to one of claims 7 or 8, wherein a number of threshold values, particularly the threshold value for the weight of the parametric models and/or the threshold for the likelihood value are adapted.

**EP 2 051 620 B1**

10. Method according to claim 1, further comprising the step of adapting the interval of autonomous evolution (2) and/or the parametric model (3) depending on external parameters (5) known to be affecting the physiological parameter (1).

11. Method according to claim 10, wherein the external parameters (5) are associated to ingestion, administration of insulin, physical activity and/or emotional stress of the patient.

12. Monitoring device for monitoring a physiological parameter (1) of a patient, in particular for monitoring a blood glucose level, comprising a physiological parameter measuring means (40) and a computing means (30), the computing means having a data storage (31) for storing measurements (4) and a processor unit (32) for calculating a prediction of the evolution of the physiological parameter, according to the method of claim 1.

13. Monitoring device according to claim 12, further comprising input means (50) for inputting external parameters (5) which are known to affect the physiological parameter (1), such as ingestion, administration of insulin, physical activity and/or emotional stress of the patient.

14. Monitoring device according to claim 13, wherein the input means comprises at least one button (51, 52, 53) for manual input of each external parameter.

15. Monitoring device according to claim 14, further comprising output means (70) for outputting alarm messages and/or advice messages to the patient.

16. Monitoring device according to claim 12, wherein the monitoring device being connected to a detector (6) for detecting a physical activity of the patient, preferably a three-axial accelerometer.

**Patentansprüche**

1. Verfahren zur Überwachung eines physiologischen Parameters (1), insbesondere zur Überwachung eines Blutzuckerspiegels, wobei das Verfahren Folgendes umfasst:

   - Protokollieren des physiologischen Parameters (1) durch wiederholte Messungen (4) und
   - Vorhersagen der Evolution des physiologischen Parameters (1),

   wobei die Evolution des physiologischen Parameters über ein Intervall autonomer Evolution (2) durch ein parametrisches Modell (3) vorhersagbar ist, wobei die Vorhersage die Folgenden Schritte umfasst:

   - Hinzufügen einer neuen Messung (4) des physiologischen Parameters zu den vorhergehenden Messungen (20) des Intervalls autonomer Evolution (2),
   - Durchführen eines Tests (21), ob die neue Messung zusammen mit den vorhergehenden Messungen immer noch ein Intervall autonomer Evolution bildet, und abhängig von diesem Test
   - Anpassen des Intervalls autonomer Evolution und vorzugsweise Anpassen des parametrischen Modells (3),
   - Vorhersagen (7) der Evolution des physiologischen Parameters anhand des parametrischen Modells (3) für das angepasste Intervall autonomer Evolution und
   - Zuordnen eines Likelihood-Wertes zu der vorhergesagten Evolution des physiologischen Parameters, wobei die Vorhersage der Evolution des physiologischen Parameters ausgesondert (23) wird, wenn der Likelihood-Wert unter einem bestimmten Schwellenwert liegt.

2. Verfahren nach Anspruch 1, wobei das Intervall autonomer Evolution (2) angepasst wird, indem mindestens eine Messung (22) der Vielzahl von Messungen des Intervalls autonomer Evolution entfernt wird.

3. Verfahren nach Anspruch 2, wobei der Test durchgeführt wird, ob die neue Messung zusammen mit den Messungen des angepassten Intervalls ein Intervall autonomer Evolution bildet.

4. Verfahren nach Anspruch 3, wobei die Schritte des Entfernens mindestens einer Messung (22) und des Durchführens des Tests wiederholt werden, bis das angepasste Intervall ein Intervall autonomer Evolution ist.

5. Verfahren nach einem der Ansprüche 1 oder 3, wobei der Schritt des Durchführens des Tests Folgendes umfasst.

- Berechnen eines Fehlers durch Vergleichen des physiologischen Parameters der neuen Messung mit einem vorhergesagten physiologischen Parameter einer vorhergehenden Iteration, oder
- Ermitteln eines neuen Parametersatzes für das parametrische Modell, wobei das parametrische Modell mit dem neuen Parametersatz die Evolution des physiologischen Parameters über das Intervall autonomer Evolution einschließlich der neuen Messung optimal beschreibt, und Berechnen eines Abstands zwischen einem neuen Vektor, der durch den neuen Parametersatz definiert wird, und einem früheren Vektor, der durch einen Parametersatz einer vorhergehenden Iteration definiert wird.

6. Verfahren nach Anspruch 1, das weiterhin Folgendes umfasst:

- den Schritt des Alarmierens eines Patienten, falls die vorhergesagte Evolution (7) des physiologischen Parameters (1) unter oder über einem gefährlichen Niveau liegt und/oder
- den Schritt des Ausgebens einer Empfehlung, insbesondere des Vorschlagens eines zu verabreichenden Wirkstoffs oder einer zu verabreichenden Wirkstoffmenge, insbesondere einer Insulinmenge.

7. Verfahren nach Anspruch 1, wobei der Schritt des Vorhersagens der Evolution des physiologischen Parameters durch Verwenden einer Vielzahl von parametrischen Modellen für das angepasste Intervall autonomer Evolution und Erlangen der Vorhersage als eine gewichtete Summe der Vorhersagen der Vielzahl parametrischer Modelle durchgeführt wird, wobei die Gewichte der parametrischen Modelle adaptiv definiert werden, vorzugsweise abhängig von einer Qualität der Vorhersage jedes parametrischen Modells in den vorhergehenden Iterationen und/oder abhängig von der Anzahl Iterationen, die jedes parametrische Modell verwendet hat.

8. Verfahren nach Anspruch 7, wobei jedes parametrische Modell ausgesondert wird, wenn sein Gewicht unter einen Schwellenwert fällt, und ein parametrisches Ersatzmodell zu der Vielzahl parametrischer Modelle hinzugefügt wird.

9. Verfahren nach einem der Ansprüche 7 oder 8, wobei eine Anzahl von Schwellenwerten, insbesondere der Schwellenwert für das Gewicht der parametrischen Modelle und/oder der Schwellenwert für den Likelihood-Wert, angepasst werden.

10. Verfahren nach Anspruch 1, das weiterhin den Schritt des Anpassens des Intervalls autonomer Evolution (2) und/oder des parametrischen Modells (3) abhängig von externen Parametern (5) umfasst, die bekanntlich den physiologischen Parameter (1) beeinflussen.

11. Verfahren nach Anspruch 10, wobei die externen Parameter (5) der Nahrungsaufnahme, der Verabreichung von Insulin, der körperlichen Aktivität und/oder dem emotionalen Stress des Patienten zugehörig sind.

12. Überwachungsvorrichtung zur Überwachung eines physiologischen Paramters (1) eines Patienten, insbesondere zur Überwachung eines Blutzuckerspiegels, mit einem Mittel (40) zum Messen des physiologischen Parameters und einem Berechnungsmittel (30), wobei das Berechnungsmittel einen Datenspeicher (31) zum Speichern von Messungen (4) und eine Prozessoreinheit (32) zum Berechnen einer Vorhersage der Evolution des physiologischen Parameters entsprechend dem Verfahren nach Anspruch 1 umfasst.

13. Überwachungsvorrichtung nach Anspruch 12, weiterhin mit Eingabemitteln (50) zum Eingeben externer Parameter (5), die bekanntermaßen den physiologischen Parameter (1) beeinflussen, wie die Nahrungsaufnahme, die Verabreichung von Insulin, körperliche Aktivität und/oder emotionaler Stress des Patienten.

14. Überwachungsvorrichtung nach Anspruch 13, wobei das Eingabemittel mindestens eine Taste (51, 52, 53) für die manuelle Eingabe jedes externen Parameters umfasst.

15. Überwachungsvorrichtung nach Anspruch 14, weiterhin mit Ausgabemitteln (70) zum Ausgeben von Alarmmeldungen und/oder Empfehlungsmeldungen an den Patienten.

16. Überwachungsvorrichtung nach Anspruch 12, wobei die Überwachungsvorrichtung mit einem Detektor (6) zum Detektieren einer körperlichen Aktivität des Patienten, vorzugsweise einem dreiachsigen Beschleunigungsaufnehmer, verbunden ist

**Revendications**

1.  Procédé pour surveiller un paramètre physiologique (1), en particulier pour surveiller un niveau de glucose dans le sang, consistant à

    - enregistrer le paramètre physiologique (1) grâce à des mesures répétées (4) et
    - prédire l'évolution du paramètre physiologique (1),

    dans lequel l'évolution du paramètre physiologique sur un intervalle d'évolution autonome (2) est prévisible grâce à un modèle paramétrique (3), la prédiction comprenant les étapes consistant à

    - ajouter une nouvelle mesure (4) du paramètre physiologique aux mesures précédentes (20) de l'intervalle d'évolution autonome (2),
    - effectuer un essai (21), pour savoir si la nouvelle mesure conjointement avec les mesures précédentes forment toujours un intervalle d'évolution autonome et, en fonction de cet essai,
    - adapter l'intervalle d'évolution autonome et adapter de préférence le modèle paramétrique (3),
    - prédire (7) l'évolution du paramètre physiologique, au moyen du modèle paramétrique (3), pour l'intervalle adapté d'évolution autonome et

    associer une valeur de vraisemblance à l'évolution prédite du paramètre physiologique, la prédiction de l'évolution du paramètre physiologique étant écartée (23) si la valeur de vraisemblance se trouve en dessous d'une certaine valeur de seuil.

2.  Procédé selon la revendication 1, dans lequel l'intervalle d'évolution autonome (2) est adapté en supprimant au moins une mesure (22) d'une pluralité de mesures de l'intervalle d'évolution autonome.

3.  Procédé selon la revendication 2, dans lequel l'essai est effectué si la nouvelle mesure conjointement avec les mesures de l'intervalle adapté forment un intervalle d'évolution autonome.

4.  Procédé selon la revendication 3, dans lequel les étapes de suppression d'au moins une mesure (22) et de réalisation de l'essai sont répétées jusqu'à ce que l'intervalle adapté soit un intervalle d'évolution autonome.

5.  Procédé selon l'une quelconque des revendications 1 ou 3, dans lequel l'étape de réalisation de l'essai consiste à

    - calculer une erreur en comparant le paramètre physiologique de la nouvelle mesure à un paramètre physiologique prédit d'une itération précédente, ou
    - déterminer un nouvel ensemble de paramètres pour le modèle paramétrique,

    dans lequel le modèle paramétrique avec le nouvel ensemble de paramètres décrit de manière optimale l'évolution du paramètre physiologique par rapport à l'intervalle d'évolution autonome comprenant la nouvelle mesure et le nouveau calcul d'une distance entre un nouveau vecteur, défini par le nouvel ensemble de paramètres et un ancien vecteur, défini par un ensemble de paramètres d'une itération précédente.

6.  Procédé selon la revendication 1, comprenant en outre

    - l'étape consistant à avertir un patient dans le cas où l'évolution prédite (7) du paramètre physiologique (1) est en dessous ou au-dessus d'un niveau dangereux et/ou
    - l'étape consistant à produire un conseil, en particulier, à suggérer un agent ou une quantité d'un agent à administrer, en particulier, une quantité d'insuline.

7.  Procédé selon la revendication 1, l'étape de prédiction de l'évolution du paramètre physiologique étant effectuée au moyen d'une multitude de modèles paramétriques pour l'intervalle adapté de l'évolution autonome et en obtenant la prédiction sous forme d'une somme pondérée des prédictions de la multitude des modèles paramétriques, dans lequel les poids des modèles paramétriques sont définis de manière adaptative, de préférence en fonction d'une qualité de la prédiction de chaque modèle paramétrique dans les itérations précédentes et/ou en fonction du nombre d'itérations que chaque modèle paramétrique a utilisé.

8.  Procédé selon la revendication 7, dans lequel chaque modèle paramétrique est écarté lorsque son poids tombe en dessous d'une valeur de seuil et un modèle paramétrique de remplacement est ajouté à la multitude de modèles paramétriques.

9. Procédé selon l'une quelconque des revendications 7 ou 8, dans lequel un nombre des valeurs de seuil, en particulier, la valeur de seuil pour le poids des modèles paramétriques et/ou le seuil pour la valeur de vraisemblance sont adaptés.

10. Procédé selon la revendication 1, comprenant en outre l'étape consistant à adapter l'intervalle d'évolution autonome (2) et/ou le modèle paramétrique (3) en fonction des paramètres externes (5) connus pour affecter le paramètre physiologique (1).

11. Procédé selon la revendication 10, dans lequel les paramètres externes (5) sont associés à l'ingestion, l'administration d'insuline, l'activité physique et/ou la tension émotionnelle du patient.

12. Dispositif de surveillance pour surveiller un paramètre physiologique (1) d'un patient, en particulier, pour surveiller un niveau de glucose dans le sang, comprenant des moyens de mesure de paramètre physiologique (40) et des moyens de calcul (30), les moyens de calcul ayant une mémoire de données (31) destinée à stocker des mesures (4) et une unité de traitement (32) destinée à calculer une prédiction de l'évolution du paramètre physiologique, selon le procédé de la revendication 1.

13. Dispositif de surveillance selon la revendication 12, comprenant en outre des moyens d'entrée (50) destinés à entrer les paramètres externes (5) qui sont connus pour affecter le paramètre physiologique (1), tels que l'ingestion, l'administration d'insuline, l'activité physique et/ou la tension émotionnelle du patient.

14. Dispositif de surveillance selon la revendication 13, dans lequel les moyens d'entrée comprennent au moins un bouton (51, 52, 53) pour entrer manuellement chaque paramètre externe.

15. Dispositif de surveillance selon la revendication 14, comprenant en outre des moyens de sortie (70) pour produire des messages d'alarme et/ou des messages de conseil au patient.

16. Dispositif de surveillance selon la revendication 12, dans lequel le dispositif de surveillance est connecté à un détecteur (6) pour détecter une activité physique du patient, de préférence un accéléromètre tri-axial.

FIG. 1

FIG. 2

FIG. 3

**FIG. 4**

**FIG. 5**

**EP 2 051 620 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

US 6923763 B1